# EUROPEAN PATENT APPLICATION

(11) **EP 4 732 796 A2**
(43) Date of publication of application: **29.04.2026**
(21) Application number: 25210383.3
(22) Date of filing: 22.10.2025
(51) Int. Cl.: A61B 17/88, B25B 21/00, B25B 23/00, B25B 23/14

(54) **TORQUE LIMITER AND COUNTER TORQUE DEVICE AND SYSTEM**

(30) Priority: 22.10.2024 US 202463710233 P
(71) Applicant: C-TORQ, LLC, Moreland Hills, Ohio 44022 (US)
(72) Inventor: Nilsson, Carl Michael, 44022 Ohio (US)
(74) Representative: IK-IP LTD

(57) **Abstract**

A device applies torque to a target component and applies counter-torque to a base component while the target component is rotated with regard to the base. The device may include a power source portion configured to generate torque and an engagement portion at a distal end of the device. The engagement portion may be selectably removable from the power source portion and includes a rotating shaft that engages a target component and is disposed within a housing having an engagement tip. The engagement tip may be configured to engage a target base. A torque limiter is removably positioned between the power source portion and the engagement portion and couples a powered distal tip of the power source portion and the rotating shaft. The torque limiter prevents a transfer of torque between the powered distal tip and the rotating shaft when the transferred torque is greater than a calibrated torque value.

## Description

### BACKGROUND

A driver-type tool can be used to drive a fastener into (or out of) a target component. When driving the fastener into the target component torque is applied to the fastener by the driver, and counter-torque can be applied to the driver by a user (e.g., manually). When torque is applied to the fastener counter-torque is applied to the driver and/or the target component in order to allow the fastener to drive into the component (e.g., due to friction, etc.), otherwise either the target component will rotate with the fastener, or the driver will rotate in the intended rotational direction of the shaft. Typically, the counter-torque needed must be applied by the user with a separate device and/or some type of external stabilizing frame.

Further, it may be desirable for the driver-type tool to apply torque to the fastener to a specific or target torque specification or value. Applying a torque to the fastener below this value (undertorqueing the fastener) may result in the fastened components becoming loose and disengaged from each other. Applying a torque to the fastener over this target value(overtorquing the fastener), can result in damage to the fastener or joint, like stripping the thread or damaging/cracking the joint materials, which may also compromise the joint and cause a failure.

### SUMMARY

This Summary is provided to introduce a selection of concepts in a simplified form that are further described below in the Detailed Description. This Summary is not intended to identify key factors or essential features of the claimed subject matter, nor is it intended to be used to limit the scope of the claimed subject matter.

One or more techniques and systems are described herein for a device that applies torque to a target component and applies counter-torque to a base component while the target component is rotated with regard to the base. The device may include a power source portion at a proximal end of the device, the power source portion configured to generate torque and an engagement portion at a distal end of the device. The engagement portion may be selectably removable from the power source portion and include a counter-torque tube, a rotating shaft disposed within the counter-torque tube and configured to operably engage a target component, and an engagement tip at the distal end of the counter-torque tube. The engagement tip may be configured to engage the base. A torque limiter may be removably positioned between the power source portion and the engagement portion and may operably couple a powered distal tip of the power source portion and the rotating shaft. The torque limiter may be configured to prevent a transfer of torque between the powered distal tip and the rotating shaft when the transferred torque is greater than a calibrated torque value.

In one implementation, the torque limiter is configured to be a single-use component that is disposed of after use in the device.

In one implementation, the torque limiter includes an input member and an output member, the input member axially received within a cavity of the output member, an outer wall of the input being configured to radially engage a cavity wall of the cavity.

In one implementation, the torque limiter further including a fastener configured to axially align and draw the input member and output member together, wherein fastening and loosening the fastener changes the calibrated torque value.

In one implementation, the input member includes a plurality of input ridges equally distributed around the input member and extending outward and configured to engage a plurality of output ridges equally distributed around the cavity wall and extending inward from the cavity wall.

In one implementation, each input ridge includes an increasing ramp, a plateau, and a decreasing ramp and each input ridge includes an increasing ramp, a plateau, and a decreasing ramp.

In one implementation, the torque limiter may include an output extension extending from a distal end of the torque limiter, the output extension including a groove that extends radially inward toward a center axis of the torque limiter, the groove defining a mechanical failure point if the applied torque exceeds the calibrated torque by a predetermined threshold value.

In one implementation, the engagement portion further includes a display window that extends through a housing of the engagement portion and is configured to display an indicator on the torque limiter to provide visual confirmation of the calibrated value of the torque limiter.

In one implementation, the engagement tip may have a groove extending through a wall of the engagement tip, a load bearing edge is configured to engage a base component during fastening, the groove having a load-bearing edge that is angled away from a centerline of the groove.

In one implementation, the device includes a motor and a controller that is received within a handle of the power source portion. The motor may generate torque and the controller can be configured to calculate an applied torque based on a current draw of a battery that powers the device and reduce a speed of the motor when the applied torque approaches the calibrated torque of the torque limiter.

In one implementation, the device includes a motor and a controller that is received within a handle of the power source portion. Th motor configured to generate torque and the controller can be configured to calculate an applied torque based on a current draw of a battery that powers the device and compare the calculated torque to an expected torque to determine if the target component is crossthreaded with the target base.

In one implementation, the device may include a grip that is coupled to a handle of the power source portion with a hinge, wherein the grip can move between a deployed position and a folded position, wherein when in the deployed position, the grip extends radially outward from the handle and the device has a pistol-grip configuration.

In one implementation, the device may include a selectably attachable grip such that when the grip is secured to a handle of the power source portion the grip extends radially outward from the handle and the device has a pistol-grip configuration.

In one implementation, the engagement tip is selectably removable from the counter-torque tube.

In one implementation, the torque limiter is retained on the distal end of the handle with a retaining device, and a separate removal tool can remove the torque limiter, the removal tool configured receive the torque limiter and engage the torque limiter such that a user can withdraw the use the removal tool to overcome the retaining device and remove torque limiter from the distal end of the handle.

In one implementation, the torque limiter comprises, a cover plate, an input plate, and an output plate, the cover plate, input plate and output plate being axially aligned, wherein a resilient member is disposed between the cover plate and a top surface of the input plate to bias a bottom surface of the input plate into axially engagement with a top surface of the output plate.

In one implementation, the input plate comprises a plurality of input ridges equally distributed around the input plate and extending downward from the bottom surface of the input plate, the plurality of input ridges configured to engage a plurality of output ridges equally distributed around the output plate and extending upward from a top surface of the output plate.

One or more techniques and systems are described herein for a torque limiter for use in a device that applies torque to a target component and applies counter-torque to a base component while the target component is rotated with regard to the base, the torque limiter comprising, an input member configured to receive an input torque and an output member, the input member axially received within a cavity of the output member, an outer wall of the input being configured to radially engage a cavity wall of the cavity, wherein physical engagement of the input member and the output member determines a calibrated torque value and wherein the input member is configured to move relative to the output member to prevent a transfer of torque between the input member and the output member when an applied torque value exceeds the calibrated torque calibrated.

In one implementation, the torque limiter further includes a fastener configured to axially align and draw the input member and output member together, wherein fastening and loosening the fastener changes the calibrated torque value.

In one implementation, the input member includes a plurality of input ridges equally distributed around the input member and extending outward and configured to engage a plurality of output ridges equally distributed around the cavity wall and extending inward from the cavity wall.

In one implementation, the torque limiter includes an output extension extending from a distal end of the torque limiter, the output extension including a groove that extends radially inward toward a center axis of the torque limiter, the groove defining a mechanical failure point if the applied torque exceeds the calibrated torque by a predetermined threshold value.

One or more techniques and systems are described herein for a torque calibration verification plate configured to verify the calibration of a torque limiter, the plate comprising a first row including a plurality of alternating first torque-off screws and fences, the fences configured to simulate a target base that receives counter torque during a fastening operation that are coupled to the plate, a second row including a plurality of alternating second torque-off screws and fences, wherein the first and second torque screws are configured to engage an engagement tip of a device that is configured to apply torque to a target component and applies counter-torque to a base component while the target component is rotated with regard to the base torque, the device including a torque limiter having a calibrated torque value and the torque-off screws are configured to fail if an applied torque from the device exceeds a bottom threshold value of a tolerance range of the calibrated torque of the torque limiter, and wherein the second torque-off screws are configured to withstand the applied torque at the calibrated torque value.

In one implementation, the torque-off screws include a groove that extends into a body between a top and a bottom of the torque-off screws, the groove defining a failure point of the first torque off screws.

In one implementation, the plate includes a first area including the first row and the second row and a second area including an additional first row and an additional second row, wherein the first area is configured to verify the calibration value of the torque limiter before a surgical procedure and the second area is configured to verify the calibration value of the torque limiter after a surgical procedure.

To the accomplishment of the foregoing and related ends, the following description and annexed drawings set forth certain illustrative aspects and implementations. These are indicative of but a few of the various ways in which one or more aspects may be employed. Other aspects, advantages, and novel features of the disclosure will become apparent from the following detailed description when considered in conjunction with the annexed drawings.

### BRIEF DESCRIPTION OF THE DRAWINGS

FIGURE 1 is a perspective view of an exemplary device.
FIGURE 2 is an exploded perspective view of the exemplary device.
FIGURE 3 is a schematic drawing of the exemplary device.
FIGURE 4 is a bottom perspective view of an exemplary torque limiter.
FIGURE 5 is a top perspective cross-section view of the exemplary torque limiter. limiter.
FIGURE 6 is an exploded view of the exemplary torque limiter.
FIGURE 7 is a side view of the exemplary torque limiter.
FIGURE 8 is a cross-section side view of the exemplary torque limiter.
FIGURE 9 is a top view of the exemplary torque limiter when the input ridges and the output ridges are engaged with each other to transmit torque from the input member to the output member.
FIGURE 10 is a top view of the exemplary torque limiter when the input member has rotated in the clockwise direction and the input ridges have slipped past the output ridges.
FIGURE 11 is a top view of a second exemplary torque limiter input ridges and output ridges with a different profile and the input ridges are output ridges are engaged with each other input ridges and the output ridges are engaged with each other to transmit torque from the input member to the output member.
FIGURE 12 is a top view of the engagement of an input ridge and an output ridge of the second exemplary embodiment.
FIGURE 13 is a bottom perspective view of the input member of the second exemplary embodiment.
FIGURE 14 is a perspective view of the input ridge of the input member of the second exemplary embodiment.

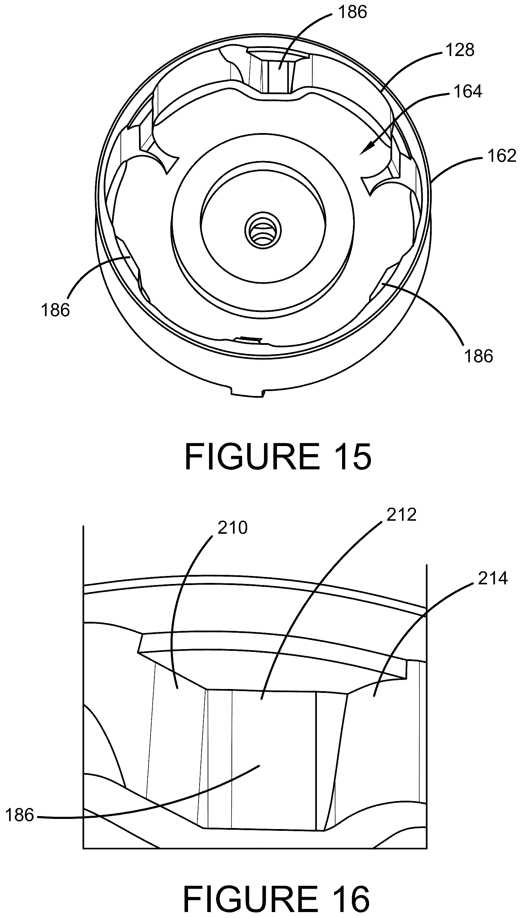

FIGURE 15 is a top perspective view of the output member of the second exemplary embodiment showing the output ridges and the cavity that receives the input member and .
FIGURE 16 is a perspective view of the output ridge of the output member of the second exemplary embodiment.
FIGURE 17 is a cross-section perspective view of an exemplary removal too with a torque limiter received within the removal tool.
FIGURE 18A shows a removal tool aligned with the distal end of the power source portion and the torque limiter engaged with the powered distal tip. FIGURE 18B shows the removal tool engaged with the torque limiter while it is still engaged with the powered distal tip. FIGURE 18C shows the removal tool removed from the distal end of the power source portion with the torque limiter retained within the removal tool.
FIGURE 19 is a perspective view of a third exemplary torque limiter that is configured as a linear actuation torque limiter.
FIGURE 20 is a cross-section perspective view of the third exemplary torque limiter.
FIGURE 21 is an exploded perspective view of the third exemplary torque limiter.
FIGURE 22 is a side view of the third exemplary torque limiter.

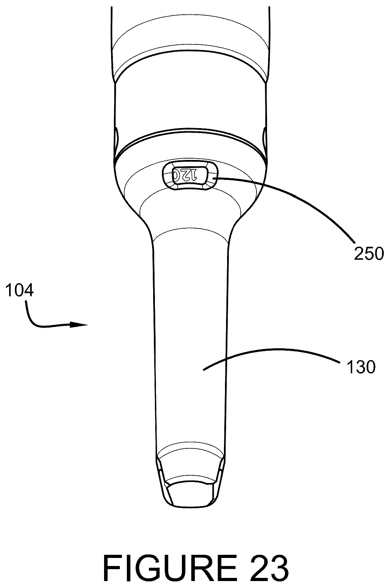

FIGURE 23 is a side view of the engagement portion of the device.
FIGURE 24A is a bottom perspective view of the distal end of the counter-torque tube and the engagement tip engaged with a target component and target base.
FIGURE 24B is a side view of an exemplary counter-torque tube engagement tip.
FIGURE 24C is a side view of the engagement tip of the counter-torque tube engaged with a target component and a target base.
FIGURE 25 is a top perspective view of an exemplary test kit.
FIGURE 26 is a top perspective view of an exemplary test kit with an engagement tip of the engagement portion engaged with a test cap in the first row of the plate.
FIGURE 27 is a top perspective cross-section view of an exemplary test kit with an engagement tip of the engagement portion engaged with a test cap.
FIGURE 28A is a top perspective view of an exemplary test cap. FIGURE 26B is a top perspective cross-section view of an exemplary test cap.
FIGURE 29A shows the device with an exemplary foldable handle in the folded position. FIGURE 29B shows the device with the exemplary foldable handle in the unfolded position.
FIGURE 30A shows the device with an exemplary removable handle engaged with the power source portion. FIGURE 30B shows the device with the exemplary removable handle disengaged from the power source portion.
FIGURE 31A is a perspective view of a target base. FIGURE 31B is a torque plot for a target based without cross-threading.
FIGURE 32A is a perspective view of a target base with a cross-threaded set screw. FIGURE 32B is a torque plot for the target based having a cross-threaded set screw.

### DETAILED DESCRIPTION

The claimed subject matter is now described with reference to the drawings, wherein like reference numerals are generally used to refer to like elements throughout. In the following description, for purposes of explanation, numerous specific details are set forth in order to provide a thorough understanding of the claimed subject matter. It may be evident, however, that the claimed subject matter may be practiced without these specific details. In other instances, structures and devices are shown in block diagram form in order to facilitate describing the claimed subject matter.

In one aspect, a device that applies torque to a tool (e.g., fastener tool, drill, other rotating tools) disposed at the end of a rotating shaft can be used to drive a target component (e.g., fastener, auger, etc.) into a target base (e.g., surface, earth, other targeted parts). Further, in this aspect, a counter-torque component can be fixedly engaged with the housing and/or handle of the device, where the counter-torque component is designed to engage with the target base. In this way, for example, the base can be stabilized by the counter-torque component to mitigate rotation of the base with the target component to which it is engaged. In this example, the stabilization of the base can provide a counter-torque that is transferred to the device housing by way of the counter-torque component.

As one example, in this aspect, the exemplary device may allow a surgeon to use merely one hand to place a screw (e.g., tighten a set screw) in a spinal stabilization device, while it drives (e.g., applies the torque to) the set screw while also holding on to the spinal stabilizer or spinal rod to apply a counter torque to the device in place. As another example, the driver can be used to tighten a nut and bolt construct (e.g., or loosened) using merely one hand by applying a tightening torque to the nut, and a counter-torque to the bolt with the same driver. As another example, this device can be used to apply torque and counter-torque to different components at the same time while mitigating stresses, moments, or torques extending beyond the device, because substantially all stresses, torques and counter-torques are contained within the device. As another example, the driver can be used to screw a fastener into a substrate, while the counter-torque is applied to the substrate, for example screwing a screw into a substrate such as, but not limited to, bone, metal, or wood. As another example, an auger may be used to draw earth from the ground, while the housing of the device is engaged with the ground to apply counter-torque to stabilize the device.

For certain uses, for instance use by a surgeon, the user may need the exemplary device to apply torque to the target and/or target base at a specific torque specification. If more torque is applied to the target than desired, i.e., the target is overtorqued, the target, target base, or substrate may be damaged or fail. If less torque is applied to the target than desired, i.e., the target is undertorqued, the target and target base may not be securely fastened together with the substrate, and the joint may fail. Ultimately, overtorquing or undertorquing the target may compromise the joint and lead to a joint failure. As such, the tool can be configured to apply the torque to a target value and/or within a tolerance range of a target value with a torque limiter. A torque verification tool can be used to verify the device is applying torque at the target value provided by the torque limiter.

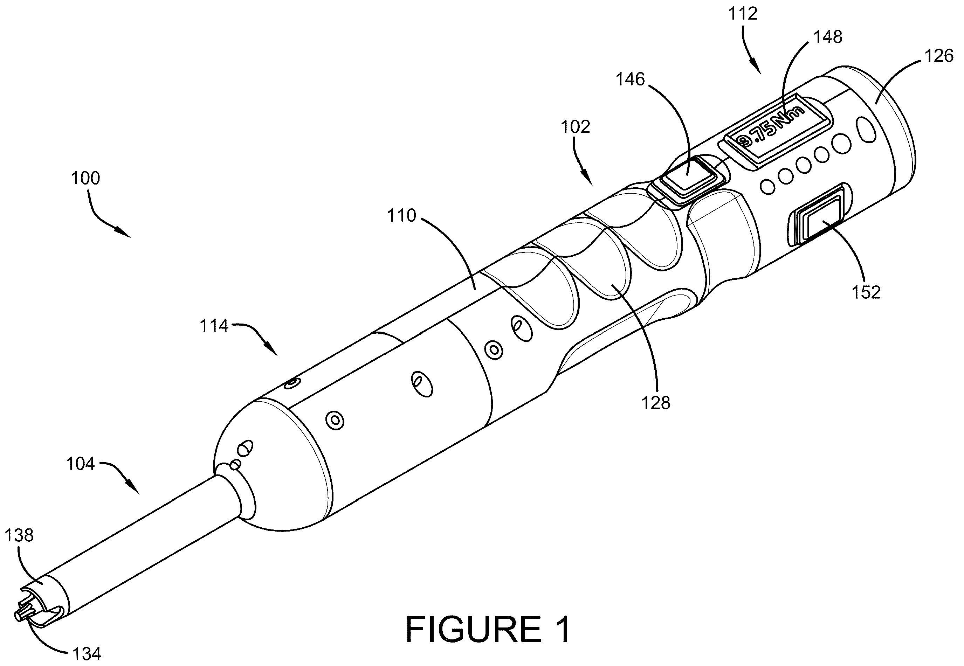

Figures 1-3 illustrate one example implementation of a device 100 for applying torque to a fastener (e.g., a threaded fastener, such as a screw or bolt) in a first direction while applying counter-torque in a second direction. In this way, for example, the threaded fastener can be driven into (e.g., or out of) a target base using the threads of the screw, while rotation of the target base and/or device 100 is mitigated.

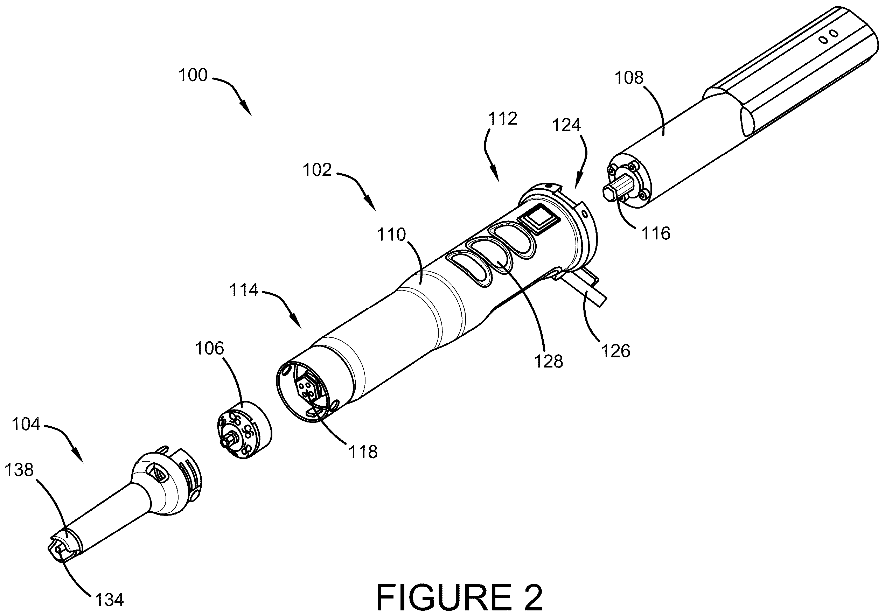

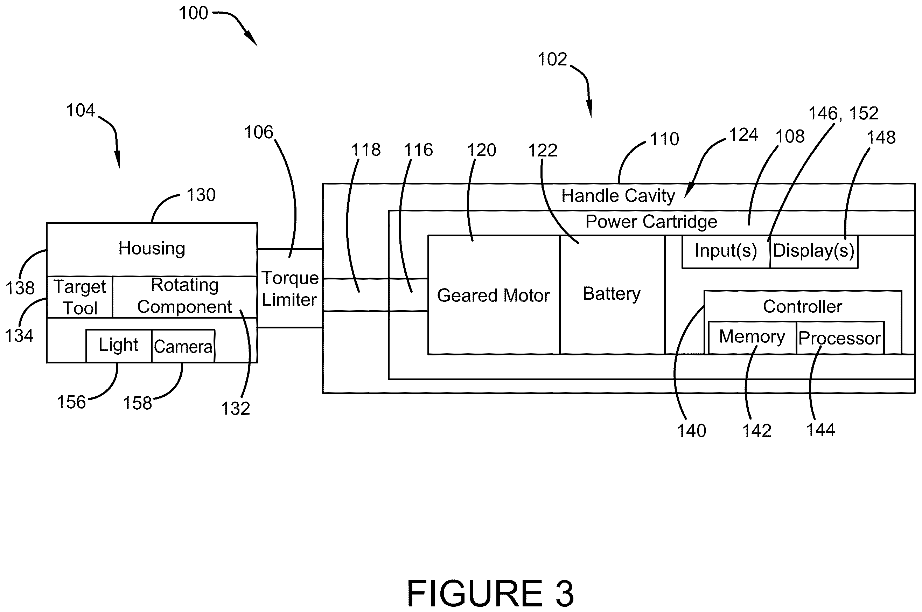

As illustrated in Figure 2, and schematically in Figure 3, the example device 100 comprises a selectably separable power source portion 102 and distal tip engagement portion 104. A torque limiter 106 can disposed between the power source portion 102 and the engagement portion 104. In some implementations, the selectably separable power source portion 102 and distal tip engagement portion 104 can be fixed together using conventional fasteners, latches, twist-lock engagement, and/or using magnets. Further, the power source portion 102 can comprise a power cartridge 108 that is received within a handle 110 having a proximal end 112 and a distal end 114. As will be discussed below, the power cartridge 108 generates the torque that is applied by the device 100. A powered tip 116 of the power cartridge 108 is operably coupled with an adapter 118 at the distal end 114 of the handle. The power cartridge 108 can be an assembly of a rechargeable battery and geared motor. The geared motor 120 and/or the battery 122 can be selectably removable from the handle 110. A cavity 124 may extend into the proximal end of the handle 110 toward the distal end 114 of the handle 110. The cavity 124 is configured to slidably receive power cartridge 108 such that the powered tip 116 engages the adapter 118 from within the cavity 124. The geared motor 120, and the battery 122 can all be combined together into one cartridge that is slidably received within the cavity 124. Alternatively, the geared motor 120 and the battery 122 may each be a separate cartridge that are slidably received within the cavity 124 of the handle 110 and operably engaged with each other. The cavity 124 can include a keyway or a slot that is configured to engaged features on the cartridge(s) to ensure proper alignment within the cavity 124. A lid 126 can cover the cavity opening when the components are installed in the cavity 124. The lid 126 can be coupled to the handle 110, for instance with a hinge. Alternatively, the lid 126 can be selectably removable from the handle 110 and can be secured to the handle 110 using conventional fasteners, latches, twist-lock engagement, and/or using magnets.

The engagement portion 104 comprises a housing 130 that houses a rotating component 132 (e.g., shaft) that drives a target tool 134 (e.g., fastener bit), which, for example, may be of any particular design configured to operably interface with the target component (e.g., fastener). The housing 130 of the engagement portion 104 may be a cylindrical tube that is configured to engage the distal end 114 of the handle 110 at a first end and is configured to engage a target component and base at the other end. The housing 130 houses the rotating component 132 such housing 130 and the rotating component 132 are coradial and the central axis of the housing 130 is coaxial with the rotational axis of the rotating component 132. The rotating component 132 may be retractable or spring loaded within the housing 130. The distal end of the housing 130 includes an engagement tip 138 that is configured to operably engage a base component or surface to provide counter-torque to the torque provided to the target tool 134 by the rotating component 132. The engagement portion 104 can be selectably swapped with different versions, with each version configured to engage with a corresponding target operation. Alternatively, the target tool 134 and/or the engagement tip 138 can be selectably swapped with different versions, with each version configured to engage with a corresponding target operation. That is, for example, a first fastener may utilize a first tip, while a second fastener may utilize a second tip. Additionally, a first engagement tip may engage with a first component or surface, while a second engagement tip may engage with a second component or surface. In this way, the engagement portion 104 can be selected for the corresponding operation. As an example, different distal tips can be designed for set screw tightening, drilling, tapping, counter-torque wrench operations, and any other rotary operation that utilizes torque and counter-torque.

When the power source portion 102 is engaged with the engagement portion 104 and the device 100 is in use, the rotational movement and torque from the geared motor 120 is transferred to the engagement portion 104 to apply torque to the target component. Specifically, the geared motor 120 drives the powered tip 116 which is engaged with a first side of an adapter 118a. A second side of the adapter 118b extends out from the distal end 114 of the handle 110 and engages and rotates the torque limiter 106. The torque limiter 106 transfers the rotational movement and torque to the rotating component 132 of the engagement portion 104. As described in further detail below, the torque limiter 106 may be configured to prevent the transfer of the motion and torque if the applied torque is over the calibrated value of the torque limiter 106. In some embodiments, a rotational engagement device (not shown) can be used as opposed to a torque limiter 106. The rotational engagement device may be the same shape and size as the torque limiter 106 and simply transfers the rotational movement between the powered tip 116 to the rotating component 132 without limiting torque. The target tool 134 engages the target component to apply torque to the target component and the engagement tip 138 engages the target base to apply counter torque to the target base.

As shown in Figures 1 and 2, the device 100 is generally configured as an inline tool, such that the handle 110 has a main axis 111 that extends along a centerline of the device longitudinally from the first end of the handle to the second end of the handle. The handle 110 may include grooves or other grip features to provide an integral grip 128 on the handle 110. To hold the device 100, an operator can wrap their hand and fingers around the grip 128 of the handle 110. The inline configuration allows an operator to hold and operate the device 100 in a more natural position than conventional pistol grip tools while using the device 100. The linear axis formed by the user's fingers and hand is coradial to the rotational axis of the geared motor 120, the rotating component 132, and the distal tip engagement 104. It will be appreciated that the handle 110 can also be designed with a handle extending out of the handle 110 such that the device is a pistol grip style device. As will be described in further detail below, the device can also include a foldable or removable grip to allow a user to selectably change the device 100 from an inline configuration to a pistol grip configuration.

As described herein, the power source portion 102 can also comprise a controller 140, which comprises memory 142 and a processor 144. The memory 142 can store data and instructions used for various operations of the device 100, and the processor 144 can be used to process the data and instructions to perform the operations. As an example, the memory 142 can store data indicative of use of the device, such as torque applied during operations, which can be downloaded for later processing and viewing. Additionally, one or more inputs 146 and display(s) 148 can be used to control functions and display information, related to the use of the device 100. In some implementations, the controller 140 can comprise an audio component that provides an audible or tactile (vibration) indicator that identifies when the torque limit has been reached (e.g., or other alerts for the user). The controller 140 can receive information from sensors to control the device 100. For instance, a strain sensor may be positioned and configured to provide data allowing the controller to determine a real-time torque value during fastening. The controller 140, memory 142, processor 144, and any other electronic components including but not limited to communications (wireless/Bluetooth/etc.) devices maybe be housed within the power cartridge 108.

The device 100 can comprise a display 148 (e.g., LCD, LED, or other functional display), which can be operably used to display feedback to the user. For example, during use, the display 148 can display the amount of torque being applied (e.g., instantaneous torque, such as in newton-meters (Nm) or other appropriate units). Further, the display 148 can display a torque limit set by the user. For example, the device 100 can have user inputs (e.g., buttons, switches, dials, widget on a touch display, etc.), such as a toggle slider switch 150, that can be used to select a torque limit. The device 100 can have a communications component (e.g., wired, wireless, RFID) that allows for a torque limit (e.g., and other data) to be loaded to the memory of the controller 140. For instance, once the torque limiter 106 is installed a tag, like a passive RFID tag, placed on or in the torque limiter 106 can be sensed by the controller 140 indicating what the target torque is. Additionally, an array of indicators 152 (e.g., LED lights) can be used to provide an indication of torque applied, such as by a number and color of lights displayed (e.g., from green to yellow to red lights, with green being low, yellow being medium, and red being high).

In some implementations, the device 100 can comprise a power/direction user input 154, such as a toggle selector switch (e.g., buttons, switches, dials, widget on a touch display, etc.) that may be used to change direction of rotation of the applied torque, between clockwise and counter-clockwise. Further, the power/direction user input 154 can be configured to control an amount of power of and/or speed of the shaft rotation. That is, in some implementations, the power/direction user input 154 can be coupled with the controller 140 to control an amount of power provided to, and/or the speed of rotation of the geared motor 120. Thus, the speed of rotation of the rotating component 132 can be controlled. In some implementations, the direction of rotation and speed of rotation can be controlled by different user inputs (e.g., different switches).

It can be beneficial to control the speed of the geared motor 120 as the device is fastening a target component, and the applied torque value approaches the final target torque value. For instance, it can be beneficial to slow the rotational speed of the geared motor 120 and therefore the speed of the rotating component 132 of the engagement end 104 as the applied torque value approached the final target torque value. This reduction in speed mitigates the transfer of any reaction forces or impulses resulting from a rapid or immediate stop of rotational movement to the user or the target component or base component. As a result, it may improve the ergonomics of the device and fastening operation. The controller 140 can reduce the speed of the geared motor as the final target value is reached. The controller 140 can determine when to begin reducing the speed based on a calculated value of the torque based on current draw from the battery 122 or from a sensed valve of the torque, for instance based on received data from a strain gauge in operably communication with the rotating component 132. Alternatively, the geared motor 120 can be selected to have a maximum torque near the target torque and the geared motor may slow down on its own without any instructions from the controller 140.

In some implementations, the device 100 can comprise an illumination component 156, such as a light, disposed at the distal end of the device 100. During operation, the illumination component 156 can be activated to illuminate a target operational area. Further, in some implementations, the device 100 can comprise an image sensor 158, such as a camera, disposed at the distal end of the device 100. In this implementation, during operation, the image sensor 158 can be activated to generate images (e.g., photos, video) of the target operational area.

In some implementations, the power source portion 102 can be configured to be aseptically cleaned after use (e.g., after a surgical operation). The power source portion 102 can be recharged (e.g., in a charging station) and reused. The handle 110 of the device power source portion 102 can sterilized after use without the power cartridge 108 and then reused. Further, the torque limiter 106 can be aseptically cleaned after use, and reused, or may be disposable, with a new limiter used in a subsequent operation. Additionally, the engagement portion 104 can be sterilized, such as by autoclaving, after being separated from the power source portion 102. Alternatively, the engagement portion 104 may be disposable and replaced after use. It will be appreciated that some of the components of the device may be sterilizable and reused while other components may be one-time use components. For instance, the handle 110 and the components slidably received within the housing can be sterilized and reused, while the engagement portion 104 and the torque limiter may be used once and disposed after one use.

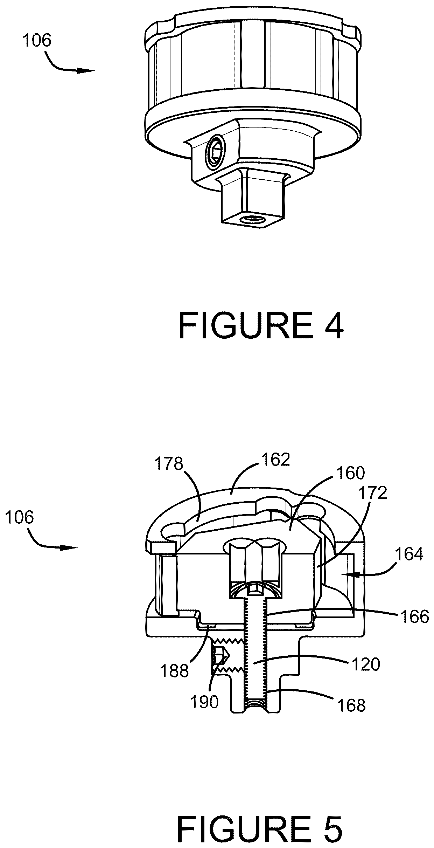

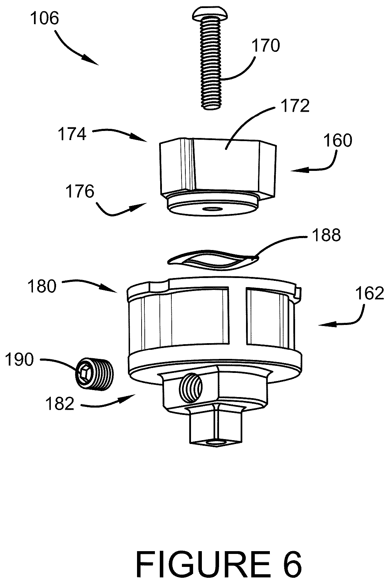

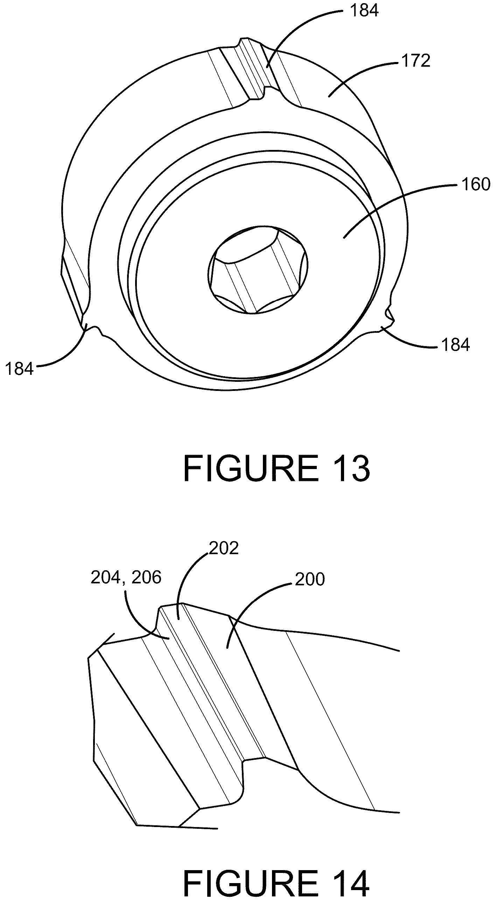

To prevent the device 100 from applying torque to a target component that exceeds a target torque, the torque limiter 106, as illustrated in Figures 4-14, can be included in the device 100. In one implementation, the torque limiter 106 can be positioned in the distal end 114 of the handle 110 of the power source portion 102. The torque limiter 106 includes a two-component assembly including an input member 160 and an output member 162. The input member 160 is received within a cavity 164 of the output member 162. The torque limiter 106 may include an optional lid component (not shown) and its inclusion or absence may not affect the provision of the target torque level. A first bore 166 extends through the input member 160 along a central axis of the input member 160, and a second bore 168 extends through the output member 162 along a central axis of the output member 162. When the input member 160 is received within the cavity 164, the first bore 166 and the second bore 168 align and are configured to receive a mechanical fastener 170 to retain the input member 160 in the cavity 164 and maintain the axial alignment of the input member 160 and the output member 162.

The torque limiter 106 generally functions from mechanical interference between portions of the input member 160 and portions of the output member 162. An outer wall 172 of the input member is angled or tapered from a larger proximal end 174 to a smaller distal end 176, such that the input member 160 has a general frustoconical shape. The cavity wall 178 of the output member 162 is angled or tapered from a larger proximal end 180 to a smaller distal end 182. The angle of the tapered walls can be selected by sound engineering judgement. When the input member 160 is received within the cavity 164 of the output member 162, the outer wall 172 of the input member 160 and the cavity wall 178 of the output member 162 form an angular (e.g., tapered or conical) clearance such that advancement of the input member 160 axially into the output member 162 by reduces the clearance between the outer wall 172 and cavity wall 178.

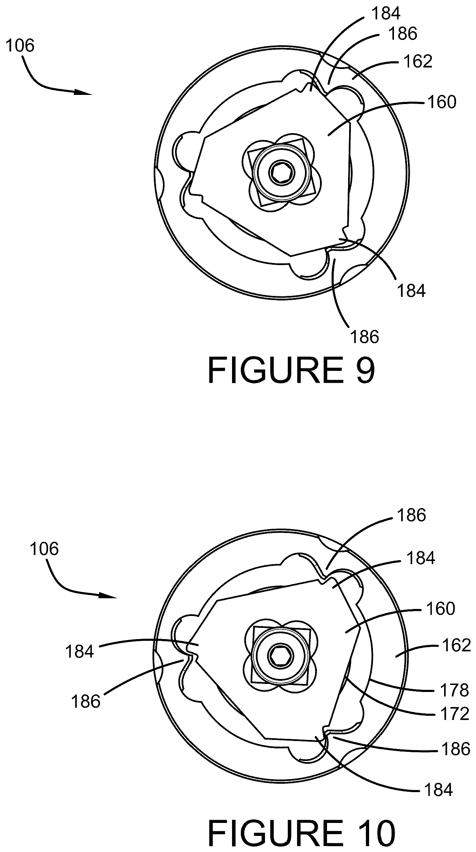

In addition to the tapered walls, the outer wall 172 includes a plurality of circumferentially distributed input ridges 184 that are configured to engage with a plurality of circumferentially distributed and corresponding output ridges 186 that extend inward from the cavity wall 178. The input ridges 184 and output ridges 186 can be ramps, facets, or teeth. As illustrated Figures 9-11, the input member 160 includes three input ridges 184 that correspond with and engage three output ridges 186 on the cavity wall 178. It will be appreciated that any number of input ridges 184 and any number of output ridges 186 can be used based on sound engineering judgement. The target torque value of the torque limiter 106 is determined by the interference of the outer wall 172 and cavity wall 178 at a controlled angle and the engagement of the input ridges 184 and the output ridges 186. When the input member 160 is rotated by the powered tip 116 through the adapter 118, the inner member 160 pushes the outer member 162 until the torque overcomes the interaction between the input ridges 184 and the output ridges 186 and the cavity wall 178 elastically deforms to allow the input ridges 184 to slip past the output ridges 186.

Once the input ridges 184 slips past the output ridges 186, the output member returns to an undeformed state. As the mechanical fastener 170 is fastened the input member 160 and output member 162 are drawn together, and when the mechanical fastener 170 is loosened the input member 160 and output member 162 can move further apart. The torque value is thus determinable and calibratable by the axial position set via the mechanical fastener 170, with greater insertion depth producing a proportionally higher torque limit from an increase in friction and interference.

In certain embodiments, the torque limiter 106 further includes a resilient member 188 disposed within the cavity 164 between the distal end 176 of the input member 160 and the bottom of the cavity of the output member 62. The resilient member 188 bias the input member 160 axially away from the output member 162 so as to maintain positional stability and prevent rattle or free play when the cooperating ridges are not in mutual engagement. As illustrated, the resilient member 188 is a wave spring washer. Alternatively, the resilient member 188 can be compression spring, disc springs, a rubber component, or some other suitable elastic member. The wave spring washer 188 may provide positional biasing only and may not be configured to affect, determine, or otherwise alter the calibrated torque limit. To prevent unintended rotation or drift of the mechanical fastener 170 during handling or use, a locking feature 190 may be provided in the form of a small set screw oriented to engage the mechanical fastener 170 after calibration from the side near the distal end of the output member 162. The locking feature 190 serves solely as a mechanical lock and likewise does not contribute to, or change, the torque limit established by the axial position set during calibration.

In some designs, the torque limiter 106 may experience kickback, which is rotational movement of the output member 162 in the opposite direction from the rotational direction during the applied torque. Kick back can arise from the input member 160 and output member 162 pushing against each other in the opposite direction after reaching a constant or stationary region once the input ridges 184 of the input member 160 slip past the output ridges 186 of the member 162. Alternatively, kickback can occur from the stored elastic energy from the deformation of the outer member 162 that allows the input ridges 184 to slip past the output ridges 186. The torque transmitted from the input member 160 to the output member 162 increases until the input ridges 184 slip past the output ridges 186. To allow the input member 162 to slip past the output ridges, the output member 162 is pushed radially outward. Once the input member 162 slips by the output member 162, the edge of the output member 162 elastically snaps back radially inward and can push the input member 162 backward, i.e., in the opposite rotational direction from the rotational direction of the applied torque.

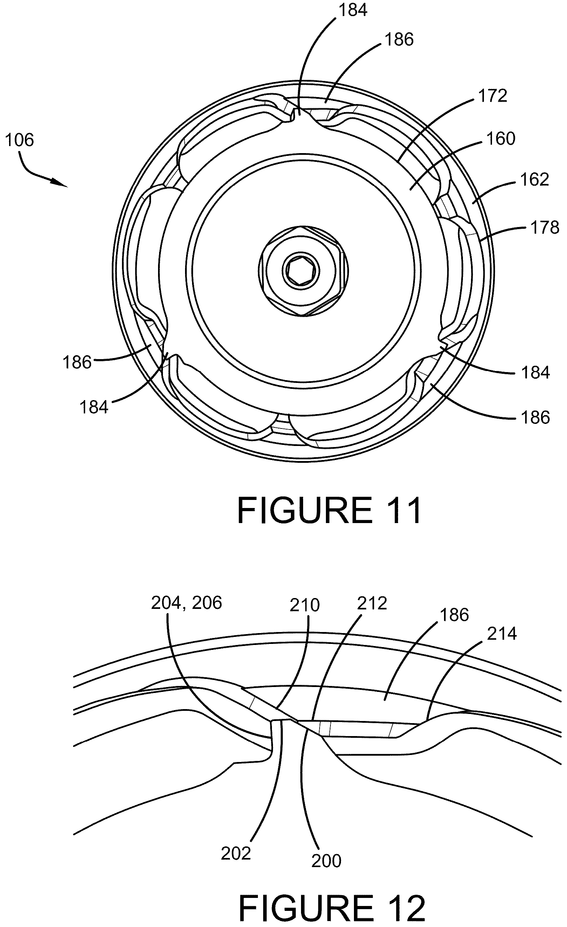

The torque limiter 106 can be designed to prevent kickback. In some embodiments, the shape and profile of the input ridges 184 and the output ridges 186 can mitigate or prevent kickback. As shown in Figures 11-14, the profile of each input ridge 184 can include an increasing ramp 200, a substantially constant plateau region 202, and a decreasing ramp 204. The profile of each output ridge 186 can include a complimentary increasing ramp 210, a substantially constant plateau region 212, and a decreasing ramp 214. The increasing ramps 200, 210 and their engagement are configured to progressively raise normal force and torque transmission as the input ridge 184 and the output ridge 186 ride up relative to each other. The substantially constant plateau regions 202, 212 and their engagement are configured to maintain a near-constant torque over a defined angular interval. The decreasing ramps 204, 214 and their engagement are configured to reduce the engagement force and thereby lower torque transfer following the constant plateau regions 202, 212. The increasing and decreasing ramps may include gradual changes in slope and/or curvature. In particular, the decreasing ramp 214 of the output ridges 186 may be formed as a gradual descent to provide a controlled reduction in torque transfer that mitigates sudden release, jerk, or kickback. Alternatively, the decreasing ramp 204 of the input ridges 184, the decreasing ramp 214 of the output ridges 186, both are formed as sharp edges or with a near-discontinuous drops, in which case torque transfer is configured to fall substantially to zero with minimal transitional decline upon reaching the edge. The exact geometry of the input ridges 184 and the output ridges 186 (including but not limited to ramp angles, step heights, and crest/root radii) can be determined through sound engineering judgement and principles to provide the gradual transmission of torque that can reduce or eliminate jerk or kickback.

In some implementations, the input ridge 184 and output ridge 186 profiles are further configured to provide defined torque-carrying capability in the reverse rotational direction (e.g., counter-tightening). The complementary sequence of the decreasing ramp 204, the constant plateau region 202, and the increasing ramp 200 of the input ridges 184 respectively acting against the decreasing ramp 214, the constant plateau region 212, and the increasing ramp 210 of the output ridges 186 establishes a reverse torque threshold T_{R}. By appropriate selection of ramp angles, plateau lengths, surface finishes/coatings, lubrication, and the axial preload set by the mechanical fastener 170, T_{R} may be intentionally tuned to be lower than, equal to, or higher than the forward torque limit T_{F}. For example, T_{R} may be selected to be less than T_{F} to facilitate controlled back-off without excessive torque. Alternatively, T_{R} may be selected to be greater than or equal to T_{F} to resist back-driving under cyclic or rebound loads. In all cases, the reverse-direction engagement of the input ridges 184 and the output ridge 186 may include a gradual or sharp decrease segment analogous to the decreasing ramps 204 and 214 to control release dynamics and mitigate unwanted jerk or kickback upon reverse disengagement.

In another embodiment, shown in Figures 9 and 10, the profile of each input ridge 184 can include an increasing ramp 200, a substantially constant plateau region 202, and a drop-off surface 206. The profile of each output ridge 186 can include a complimentary increasing ramp 210, a substantially constant plateau region 212, and a drop-off surface 216. The drop-off surfaces 206, 216 have an immediate drop-off as opposed to a gradual decrease in slope or curvature. Each drop-off surface is radial or nearly radial compared to the axis of rotation of the input member 160. In this configuration, when the input member 160 slips past the output member 162, the drop-off surfaces 206, 216 allows the deformed edge of the output member 162 to elastically snap back into an undeformed state without causing the input member 160 to kickback. This configuration may only prevent kickback from the deformed output member 162 snapping back into is undeformed state.

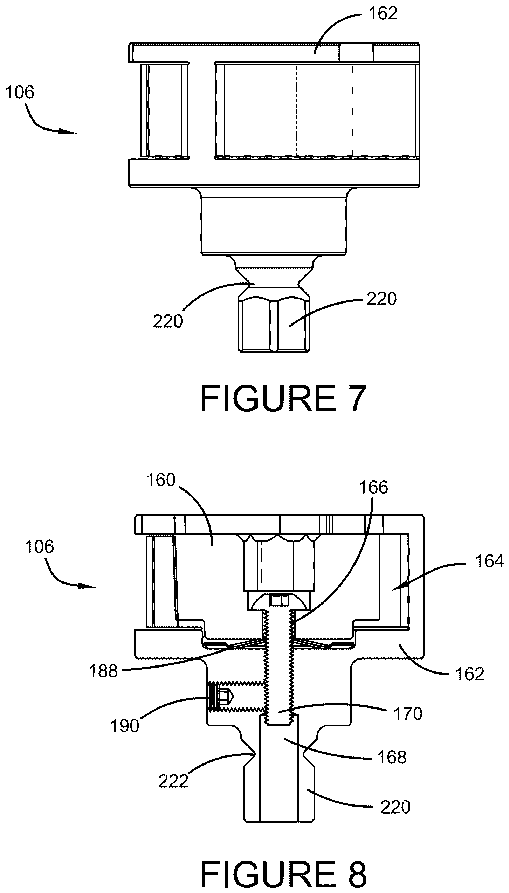

Turning to Figures 7 and 8, in certain embodiments, the torque limiter 106 may include, at its distal end, a torque output interface 220. This interface 220 may include an output extension that extends from the distal end 182 of the torque limiter 106. The output extension 220 may be a hexagonal, rectangular, or some other suitable polygonal shaped extension adapted to couple to the rotating component 132 (drive shaft) of the engagement portion 104. The output extension 220 may include a torque-off V-notch 222 that is configured to fail, for instance to shear-off or break-off, at a specified torque value that exceeds a tolerance bandwidth of the calibrated torque of the torque limiter 106. For example, the output extension 220 may be configured to fail if the torque applied by the device 100 exceeds the calibrated torque value by 3% to 10%. The V-notch 222 is dimensioned and the material of the extension 220 is selected such that the output extension 220 will fracture when the transmitted torque exceeds the max allowable torque value. Therefore, the torque limiter can include a fail-safe mechanical break to prevent torque transmission to the target beyond the intended limit while maintaining normal operation at or below the calibrated torque. It will be appreciated that the geometry, wall thickness, and notch root radius are selected according to sound engineering practices and judgment to achieve the stated calibration tolerance under expected manufacturing variations and operating temperatures.

The torque limiter 106 can be configured so that it is a single-use device component that is disposed after the torque limiter's first use and installation in the distal end 114 of the handle 110. As such, the torque-limiter 106 may be supplied sterile within a sterile barrier package that includes labeling that expressly designates the device as single-use only (i.e., intended for one procedure and thereafter to be discarded). As contemplated, multiple torque limiters 106, each having a different calibrated torque can be used in the same environment, i.e., the same surgical environment. As such, the user can select the desired output torque values that will be applied by the device during a surgical procedure by selecting different torque limiters 106. To differentiate the torque limiters, the torque limiter 106 can further include an on-device indicator 230 that indicates the torque value the torque limiter is calibrated to. The indicator 230 can be labeling comprising written alphanumeric markings, a coding scheme to identify the torque rating and lot designation, or some other indicator. The coding scheme can be color-based, shape-based, or some other suitable visual indicator/symbol that can be matched to an index of calibrated torque values. Such labeling may be implemented as permanent print, etched or molded characters, bands, or coatings located on an external surface of the torque limiter and configured to remain visible during use. In some embodiments, the color-coding is standardized across torque values to enable rapid visual identification prior to assembly and within the sterile field.

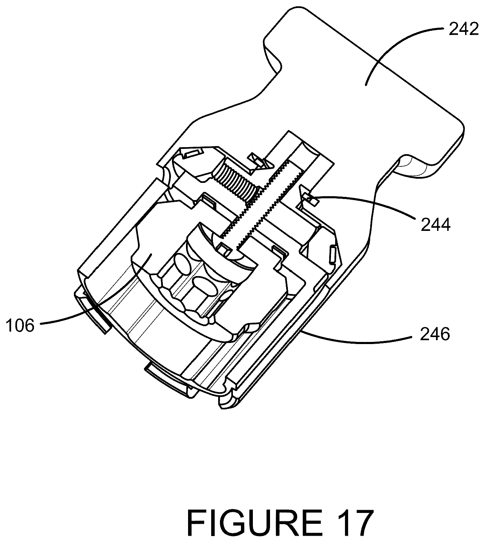

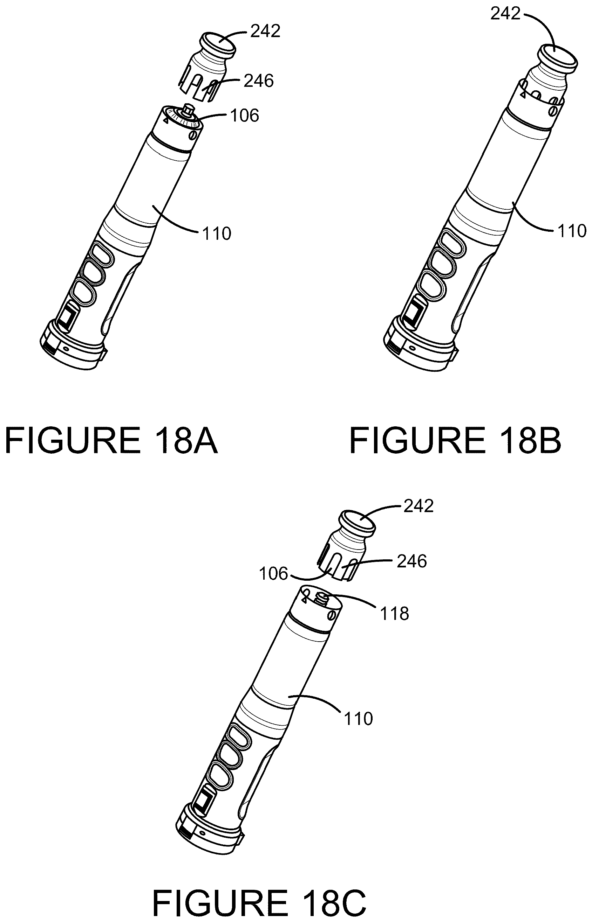

Turning to Figures 17-18C, in certain embodiments, the torque limiter 106 is configured to couple to the distal powered tip 116 of the handle 110. Once coupled to the distal powered tip 116, the torque limiter 106 may be retained in that position with a retention element 240, such as a C-ring, O-ring, spring detent, or some other suitable retention device. The retention element 240 can be dimensioned to resist unintended decoupling so that the torque limiter 106 remains on the power source portion 104 during use. To remove or decouple the torque limiter 106 from the power source portion 104 may require a dedicated removal tool 242 provided within the torque limiter's sterile packaging. The removal tool 242 may include an internal c-ring sized and positioned to snap into the torque limiter's V-notch 222. Further, the removal tool 242 can include one or more axially extending members 246 that slide along the exterior length of the torque limiter 106 and hook beneath a distal lip or undercut to provide positive axial extraction. The axially extending members 246 may substantially surround the torque limiter 106 when the removal tool 242 is engaged with the torque limiter 106 such that the torque limiter 106 is received within the removal tool 242 as shown in Figures 17 and 18C. Upon engagement, the internal C-ring 244 is configured to irreversibly engage and lock within the V-notch 222 (e.g., non-backdrivable geometry and/or interference fit), preventing the removal tool 242 from being detached from the torque limiter 106 without destruction. The removal tool 242 may be fabricated from metal or polymeric materials. Once the removal tool 242 is installed, the torque limiter 106 can be removed from the handle. However, the removal tool 242 cannot be removed from the torque limiter 106, thereby preventing re-sterilization and reuse and ensuring the torque limiter 106 is used only one-time as intended.

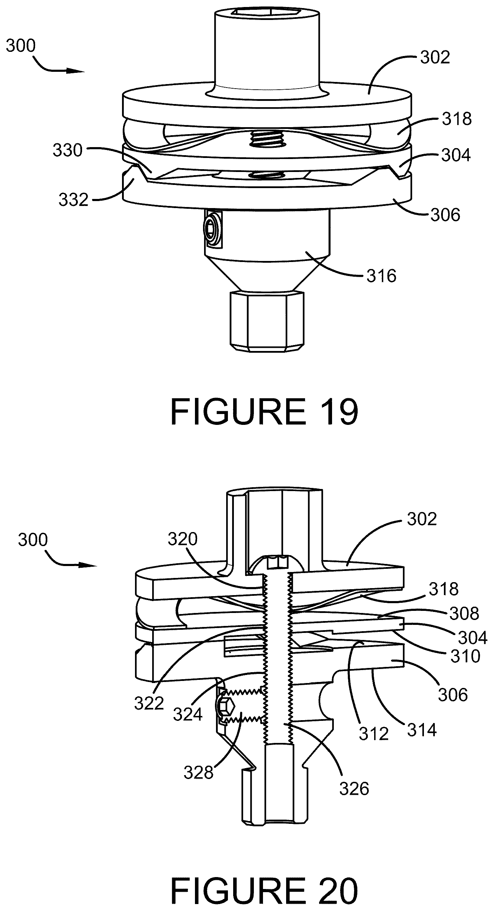

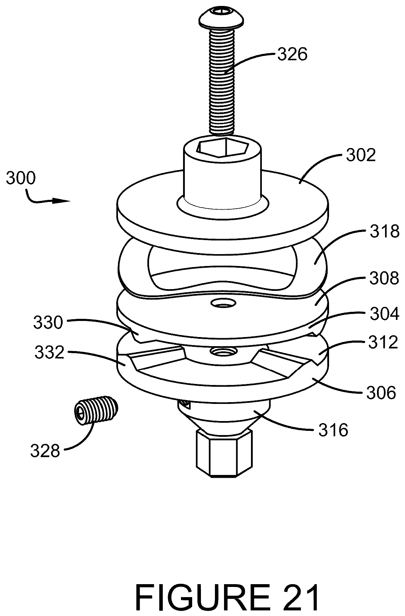

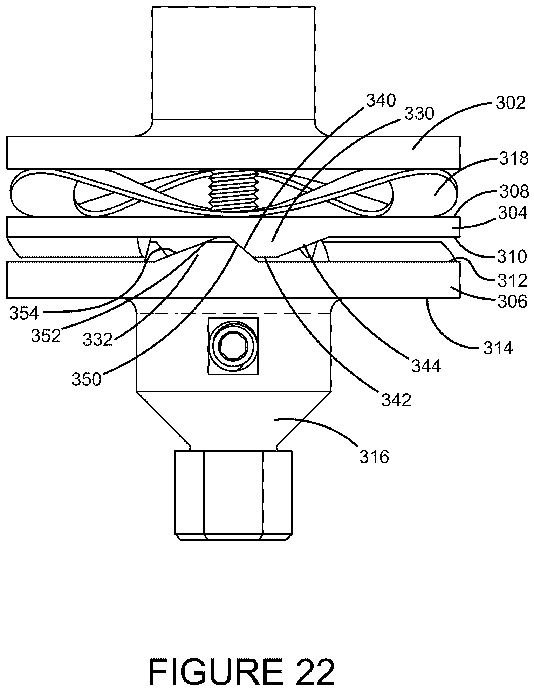

Figures 19-22 illustrate another example implementation of a torque limiter 300. As previously described, the torque limiter 106 functions based on the radial engagement of the input member 160 and the output member 162. In the alternative implementation, a linearly actuated torque limiter 300 can be used in the device 100. The linear torque limiter 300 includes a cover plate 302 that is configured to operably engage the distal powered tip 116 of the power source portion 102, an input plate 304, and an output plate 306. The bottom surface 310 of the input plate 304 is configured to engaged the top surface 312 of the output plate 306 to provide the torque limitation function. An extension 316 extends from the bottom surface 314 of the output plate 306. The extension 316 is configured to engage the rotating component 132 in the engagement portion 104 to transfer torque from the power source portion 102 to the engagement portion 104. A resilient member 318 may be disposed between the cover plate 302 and the top surface 308 of the input plate 304 to bias the input plate 304 toward the output plate 306. As illustrated, the resilient member 318 may be a wave spring washer, but any suitable resilient member can be used. A first bore 320 extends through the center of the cover plate 302, a second bore 322 extends through the center of the input plate 304, and a third bore 324 extends through the center of the output plate 306. A mechanical fastener 326, like a screw, extends through the first bore 320, the second bore 322, and the third bore 324 to draw the cover plate 302, the input plate 304, and the output plate 306 together and to maintain axial alignment of the plates. Similar to the torque limiter 106, the torque limiter 300 can be calibrated to a specific torque by fastening or loosening the mechanical fastener 326. To prevent unintended rotation or drift of the mechanical fastener 326 during handling or use, a locking feature 328 may be provided in the form of a small set screw oriented to engage the mechanical fastener 326 from the side near the distal end of the output member output plate 306 after calibration.

The input plate 304 includes a plurality of radially distributed input ridges 330 extending from the bottom surface 310 that are configured to engage with a plurality of radially distributed and corresponding output ridges 332 that extend upward from the top surface 312 of the output plate 306. The input ridges 330 and output ridges 332 can be ramps, facets, or teeth. As illustrated, the input plate 304 includes three input ridges 330 that correspond with and engage three output ridges 332 on the output plate 306. It will be appreciated that any number of input ridges 330 and any number of output ridges 332 can be used based on sound engineering judgement. The target torque value of the torque limiter 300 is determined by the interference of the input ridges 330 and the output ridges 332. When the cover plate 302 is rotated by the distal powered tip 116, the rotation is transmitted from the input plate 304 and to the output plate 306 by the engagement of the input ridges 330 and the output ridges 332. The rotation is transmitted until the torque from the joint overcomes the friction between plates and until the engagement of the input ridges 330 and the output ridges 332 overcomes the biasing force from the resilient member to force the input plate 304 upwards and the input plate 302 can slip past the output plate 306. As the mechanical fastener 326 is fastened the input plate 304 and output plate 306 are drawn together, and when the mechanical fastener 326 is loosened input plate 304 and output plate 306 can move further apart. The torque value is thus determinable and calibratable by the axial position of the plates set via the mechanical fastener 170, with greater insertion depth producing a proportionally higher torque limit from an increase spring force from the resilient member 318 and an increase in friction and between the input ridges 330 and the output ridges 332.

The torque limiter 300 may also include features to prevent or mitigate kickback. The profile of each input ridge 330 can include an increasing ramp 340, a substantially constant plateau region 342, and a decreasing ramp 344. The profile of each output ridge 332 can include a complimentary increasing ramp 350, a substantially constant plateau region 352, and a decreasing ramp 354.The relative slopes of the increasing ramps 340, 350 and the decreasing ramps 344, 354 are selectable. For instance, the increasing ramps 340, 350 may be steeper, equal, or less steep than the decreasing ramps 344, 354 so as to tune the calibrated torque threshold and release behavior while the plates ride up and over one another with controlled axial separation. Following the peak of the increasing ramps, the plateau regions 342, 352 maintain near-constant torque under elevated frictional contact. Once this frictional contact is overcome, the plates can slip relative together to prevent torque from being transmitted above the selected torque threshold value. The decreasing ramps 344, 354 can include a gradual slope or curvature transition configured to reduce engagement force in a controlled manner so as to mitigate abrupt torque drop, axial impact loads, and/or sudden reverse rotation (kickback). In an alternative embodiment, one or both decreasing ramps 344, 354 may be formed as sharp edges to effect a rapid disengagement with a near-discontinuous drop in torque transfer.

In some implementations, the input ridge 330 and output ridge 332 profiles are further configured to provide defined torque-carrying capability in the reverse rotational direction (e.g., counter-tightening). The complementary sequence of the decreasing ramp 344, the constant plateau region 342, and the increasing ramp 340 of the input ridges 330 respectively acting against the decreasing ramp 354, the constant plateau region 352, and the increasing ramp 350 of the output ridges 332 establishes a reverse torque threshold T_{R}. By appropriate selection of ramp angles, plateau lengths, surface finishes/coatings, lubrication, and the resilient member preload set by the mechanical fastener 326, T_{R} may be intentionally tuned to be lower than, equal to, or higher than the forward torque limit T_{F}. For example, T_{R} may be selected to be less than T_{F} to facilitate controlled back-off with reduced risk of over-torque. Alternatively, T_{R} may be selected to be greater than or equal to T_{F} to resist back-driving under cyclic or rebound loads. In all cases, the reverse-direction engagement of the input ridges 330 and the output ridge 332 may include a gradual or sharp decrease segment analogous to the decreasing ramps 344 and 354 to provide gradual or controlled release so as to mitigate abrupt torque drop, axial impact loads, and/or sudden reverse rotation. It will be appreciated that the torque off features of the output extension and the single-use features described above with respect to the torque limiter 106 can also be employed and used with the torque limiter 300.

Once the components of the device 100 are installed in the handle 110, and the engagement portion 104 is installed at the distal end 114 of the handle 110, a user cannot see these components. To allow a user to confirm that a correct torque limiter 106 has been selected and installed in the device 100, the housing 130 of the engagement portion 104 can include a small display window 250. The small display window 250 allows visual access to the torque limiter 106 and the indicator 230 (labeling/colors/etc.) on the torque limiter 106 described above. This indicator(s) 230 is visible through the display window. Therefore, the display window 250 allows for an additional verification step to ensure that a proper torque limiter 106 has been selected and therefore the proper torque is being applied to target component.

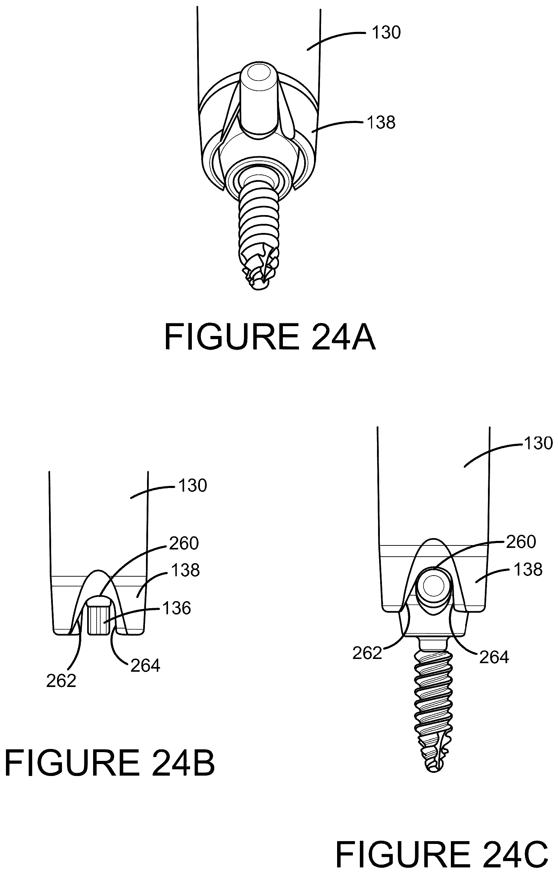

Turning to Figures 24A-24C, the engagement portion 104, particularly the engagement tip 138 can be configured to prevent binding with the target component or target base after applying torque to the target fastener/base. The engagement tip 138 can include a groove or slot 260 that extends through the walls of the tip 138. The groove or slot 260 is configured to receive and engage a part of the target component or target base For instance, the groove 260 may receive a spinal rod. The groove 260 can generally be U-shaped and has a first edge 262 and a second edge 264. Depending on the direction of rotation of the device 100 to apply torque to the target component, either the first edge 262 or the second edge 264 may be a load-bearing edge during application of torque to the target component. In conventional embodiments, the first edge and the second edge may be symmetrical and have same angle relative to the main axis of the device 100. For instance, the first edge and second edge may be parallel or almost parallel to the main axis of the device and have a relatively snug fit around the target component and/or target base. In these embodiments, the engagement tip can bind up with the target component or target base once the final torque has been applied to the target component. As a result, the device 100 may be difficult to remove from the target component or target base.

The groove 260 can be configured to prevent this binding. In this embodiment, the groove 260 may have a load-bearing edge 262 that is angled away from the main axis of the device at an anti-bind angle larger than that of a conventional counter torque device, as illustrated in Figure 24B. For instance, the anti-bind angle can range between 10° and 45° from the main axis of the device 100 and depends on the amount of torque that is needed during final tightening. The angled load-bearing edge 262 prevents any kind of binding up once final tightening has been achieved. The opposite edge 264, which is load bearing during loosening, may still have very steep angle compared to the opposite load-bearing edge during fastening. This angle can vary between 0° and 10°. Compared to the conventional counter torque device, the groove 260 of the engagement tip 138 of the engagement portion 104 engages the target component and target base enough to provide the necessary counter torque but allows for some rotational movement of the engagement portion 104 on the target component/target base. As a result, once the torque has been applied, binding will not occur and the device 100 can be removed from the target component/target base with relative ease.

Further, in some implementations, the device 100 can be configured to prevent binding with a programed torque application sequence. After the torque limit has been reached, the binding force/torque between engagement tip 138 and the target component (e.g., fastener) can be released with a very small, automatic counter rotation of the geared motor (e.g., less than 2 degrees), which does not affect the final applied torque. That is, for example, often, when applying torque to a component, the tool can be bound to the target component, which makes it difficult to remove the tool from the component without potentially adjusting the position of the component. In this implementation, the geared motor 120 can be used to automatically apply a slight counter-rotation to the engagement tip 138 to unbind the tool from the target component. In some implementations, the processor 144 in the controller 140 can be programmed to have the geared motor 120 perform a small counter-rotation (e.gt., reverses the motor) when the forward rotation activation is released (e.g., deactivated, such as at the input switch). In this way, merely enough counter-rotation can be applied to unbind the engagement portion 104 from the target component, and not undo any torque already applied to the target component. The device 100 can include both the physical anti-binding groove and the anti-binding program.

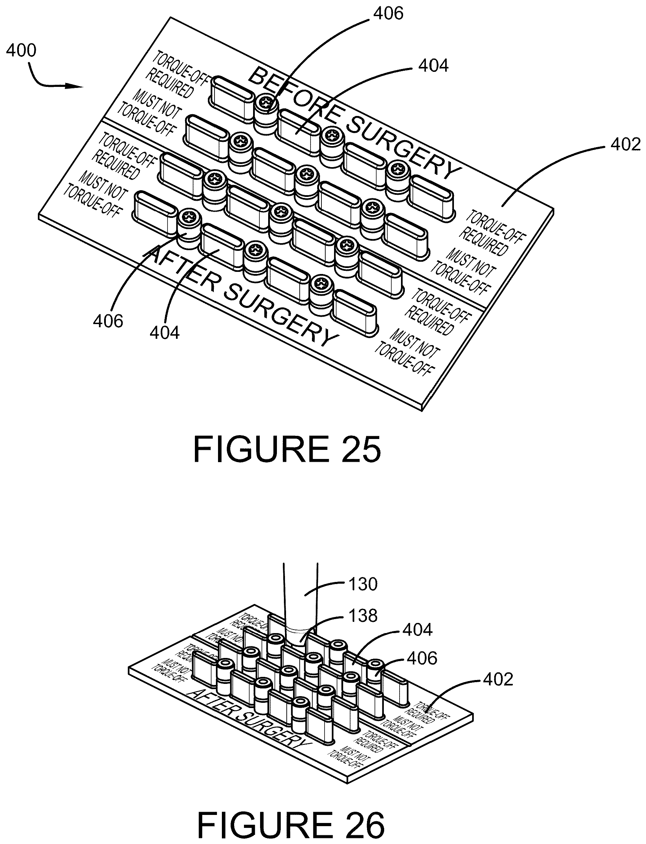

Turning to Figures 25-28B ,to verify the device 100 and/or the torque limiter are accurately applying torque to a target, the operator may use a torque verification test kit 400. The test kit consists of a metal plate 402 with fences 404 and torque-off screws 406. In one embodiment, the plate 402 has a total of four rows of at least one torque-off screw 406. The fences 404 are positioned between the torque-off screws 406 simulate a target base, like a spinal rod. As illustrated, the rows may include four fences 404 and torque-off screws 406. It will be appreciated that the number of fences 404 and screws 406 can be selected to be any number based on sound engineering and judgement. The break off / torque-off screws 406 can either be integral parts of the plate 402 or individual components that are installed on the plate 402, for instance by press-fitting a bottom 408 of the torque-off screws 406 into the plate 402.

The kit 400 may be defined for the same calibrated torque value that the toque limiter 106 is calibrated for. The kit 400 can generally function as a GO/NO-GO calibration gauge from the torque limiter 106. In some embodiments, the test kit 400 is a separate standalone item. Alternatively, the test kit 400 may be included as a part of a package having a single-use, sterile packed torque limiter 106. The plate 402 can be divided into two areas, a first area for before surgery verification and a second area after surgery verification. In an alternative embodiment, the first area and the second area can be two separate plates. Each area can include a first row of GO torque-off screws 406 that are configured to break off or fail when the device 100 applies torque to the torque-off screws 406. Each area can also include a second row of NO-GO torque-off screws 406 that do not break when the device 100 applies torque to the torque-off screws 406. As illustrated, the plate 402 can include text either printed, embossed, or engraved on the plate 402 that indicates or provides instructions for each area of the plate 402 and each row of the plate 402.

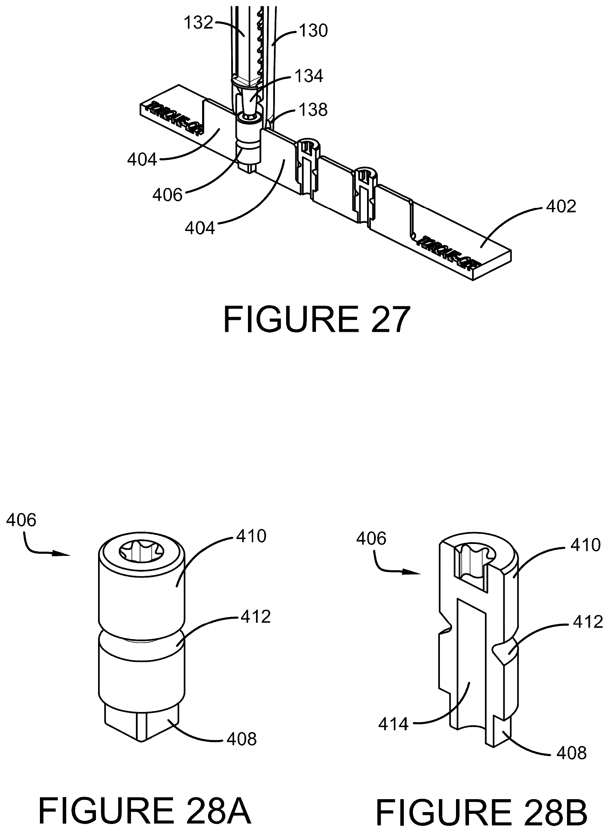

Turning to Figures 28A and 28B, the torque-off screws 406 will be described. The top 410 of the torque-off screws 406 may configured to engage the tool of the device 100, for instance, the top 410 of the torque-off screws 406 may include female drive features, like a hex drive feature configured to receive and engage the tool of the device. Between the bottom 408 and top 410 of the screws an external V-notch 412 extends into the body of the screws 406. Further, the screws 406 may include an internal hole 414 that extends up from the bottom 408 of the screws 406 such that the screw 406 is substantially hollow. The remaining material between the V-notch 412 and the internal hole 414 resists the torque applied to the torque-off screws 406 until the material fails and the torque-off screws 406 break. As such, this remaining material defines the torque required to break the torque screws 406.

For instance, the device 100 can be used with the torque limiter 106, 300 installed to verify the calibration or the torque limiter 106, 300 before applying torque to fasteners used in the surgery, for instance in the operating room before beginning surgery. Similarly, after surgery, the device 100 can be used with the torque limiter 106 installed to verify the calibration of the torque limiter 106, 300. As such, a user of the device 100 can confirm the torque limiter 106, 300 was in specification before and after the surgery. Prior to surgery, the fully assembled device 100 with the appropriate torque limiter 106, 300 installed inside is placed on the first three screws 406 in the first row and the device 100 is actuated for final tightening. All three torque-off screws 406 need to break off / torque-off (GO) in order to verify the device 100 can reach its intended torque limiting value (GO). Then the device 100 is placed on all three screws 406 in the second row and actuated for final tightening. None of the three torque-off screws 806 may break off / torque-off in order to verify the torque limiter 106, 300 does not exceed its torque limiting value (NO-GO)

After surgery, the test sequence is repeated for rows 3 and 4. If all three torque-off screws 406 in row three break off / torque-off and all three torque-off screws 406 in row four do not break off / torque-off, then the device 100 and torque limiter 106, 300 is still within the allowable range of the torque limits of the torque limiter 106. If the device 100 performs all of these steps successfully, the device performed within specifications during the surgery. If the device did not perform the above steps successfully, the device 100 and the torque limiter 106, 300 were not in specification and the fasteners that received the applied torque form the device may need to be re-torqued.

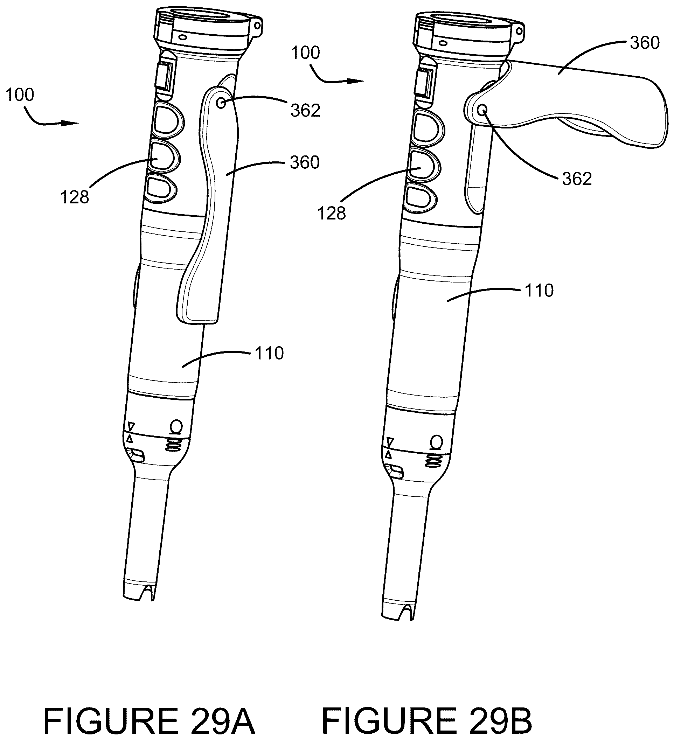

In some implementations, the device 100 can be converted into a pistol-style device. The handle 110 includes an auxiliary grip 360 mounted to the handle 110 via a hinge 362. The auxiliary grip 360 may be movable between a stowed position, shown in Figure 29A, in which it folds down against the grip 128 to preserve a generally round, in-line grasping profile, and a deployed position, showing in Figure 29B, in which the auxiliary grip 360 folds out to define a non-collinear grasping surface relative to the axis of the handle 110. The hinge 362 may incorporate a detent, over-center spring, cam, and/or snap-fit latch configured to releasably lock or bias the auxiliary grip 360 in at least the stowed and deployed positions thereby providing tactile feedback and resisting unintended movement during use. The hinge may also releasably lock the auxiliary grip in intermediate positions between the stowed and deployed positions. When in the deployed position, the auxiliary grip 360 is positioned to permit the user to grasp and stabilize the device 100 via the unfolded grip 360, improving control and ergonomics during torque application. When in the stowed position, the grip 360 lies substantially flush with the handle 110 to minimize interference and maintain the conventional cylindrical handle contour. The auxiliary grip 360 may be formed from metal and/or polymer materials and may include surface textures to enhance traction.

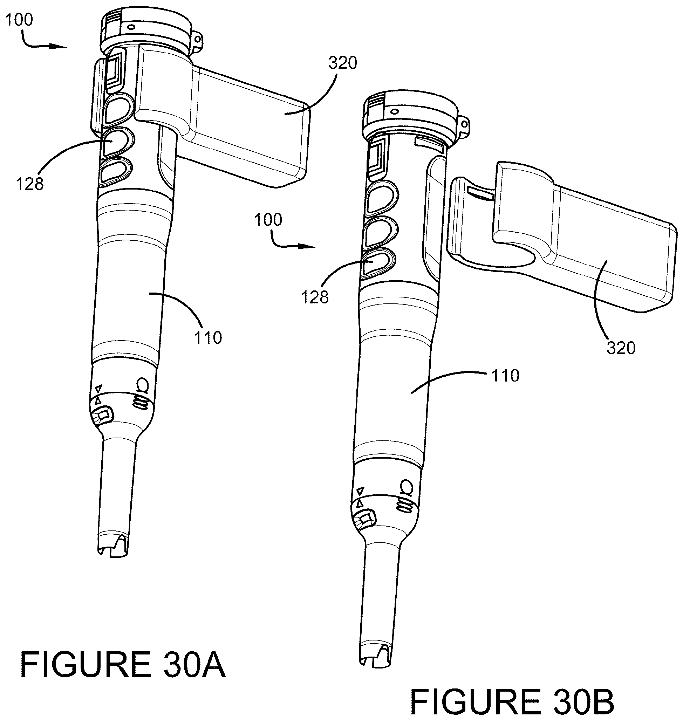

In another implementation, shown in Figures 30A and 30B, a removable auxiliary grip 370 is provided as a separate component configured to couple to the integral grip 128 in a releasable yet secure manner. The grip 370 can include features that correspond with features on the 1298 to provide a secure attachment of the grip 370 to the integral grip 128. For instance, the grip 370 may be secured to the handle 127 using a keyed interface, bayonet, snap-fit latch, threaded collar, or functionally equivalent coupling. When installed, the grip 370 establishes a non-linear (for instance, pistol-style) grasping geometry relative to the handle axis to enhance control during use. The coupling is configured to resist unintended decoupling and rotation of the grip 370 relative to the integral grip 128 under expected operational loads and vibration, thereby maintaining a firm attachment once engaged, while permitting intentional removal for use of the handle without the auxiliary grip. The integral grip 128 is fully functional in either state-without the auxiliary grip in a conventional in-line configuration, or with the auxiliary grip installed to provide an offset grasping surface. The grip 370 may include surface texturing or contours to improve traction and ergonomics.

In one implementation, the example torque/counter-torque device 100 can comprise a system that uses detected electrical use to provide feedback to a user, and/or to control the torque applied by the target tool 134 at the engagement tip 138, as described above. In this implementation, the amount of current used (e.g., drawn, as in amps) can be detected during use, and correlated to an amount of torque being applied by the device. That is, for example, empirical testing can evaluate the amount of torque applied for any given current load, and data indicative of this relationship (e.g., function) can be loaded into memory 142 of the controller 140 (e.g., control board) in the device 100. Then, during use, the processor 144 disposed on the controller 140 can detect the current load and use the stored relationship to determine the amount of torque being applied. In these implementations, this information can be used to provide feedback to the user and control the geared motor 120 of the device 100.

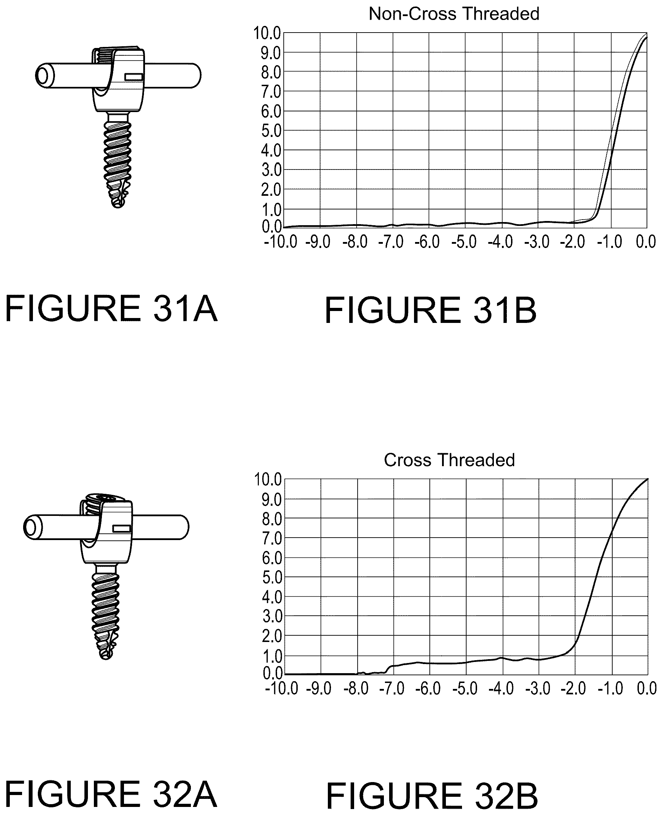

In one embodiment, the controller 140 of the device 100 can be configured to detect cross-threaded fasteners. During the final tightening of a fastener, the controller 140 can monitor the current draw on the battery 122 to monitor the applied torque at every moment. While monitoring the current draw, the controller 140 can use an algorithm or artificial intelligence to evaluate the characteristic of the current draw to determine whether the fastener is properly seated (as shown in Figure 31A) or if the fastener is cross-threaded (as shown in Figure 32A). As illustrated in Figures 31B and 32B, graphs for current draw during tightening are different for "normal" or "non-cross-threaded" and "cross-threaded" situations. The controller 140 can plot and analyze the current draw against the applied torque using different types of statistical and data analysis, including but not limited to, 1^{st} and 2^{nd} derivatives/integrals, standard deviations of multi-point averaging, etc. If the controller 140 detects a cross threaded fastener, the controller 140 can provide a notification to the user of the device 100. Based on the feedback, the user can back the fastener out and retorque the fastener or replace the fastener if necessary. As an example, the device 100 can be used to detect if the set screw of a pedicle screw is cross threaded during a surgery as shown in Figure 32A.

The device 100 can be used with a tracking or navigation system configured to track the motion and the position of objections in a surgical environment, like an operation room. As such, the device can be configured to receive or hold a tag or fiducial maker that can be sensed and tracked as the device 100 moves through the surgical environment. The marker can comprise any suitable marker like a reflective target or sphere or a plurality of markers attached to a frame. As the device 100 moves through the surgical environment, sensors, like optical sensors, can detect the position of the marker(s) to determine the position and/or orientation of the device 100. The device 100 can also be used with an automated motion system, like a surgical robot, which may automatically move and position the tool relative to a target component and target base. When used on a motion system, the device 100 can be configured to operate and apply torque remotely, i.e., without any physical interaction with physical inputs on the power source portion 102.

The word "exemplary" is used herein to mean serving as an example, instance or illustration. Any aspect or design described herein as "exemplary" is not necessarily to be construed as advantageous over other aspects or designs. Rather, use of the word exemplary is intended to present concepts in a concrete fashion. As used in this application, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. Further, at least one of A and B and/or the like generally means A or B or both A and B. In addition, the articles "a" and "an" as used in this application and the appended claims may generally be construed to mean "one or more" unless specified otherwise or clear from context to be directed to a singular form.

Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims.

Also, although the disclosure has been shown and described with respect to one or more implementations, equivalent alterations and modifications will occur to others skilled in the art based upon a reading and understanding of this specification and the annexed drawings. The disclosure includes all such modifications and alterations and is limited only by the scope of the following claims. In particular regard to the various functions performed by the above described components (e.g., elements, resources, etc.), the terms used to describe such components are intended to correspond, unless otherwise indicated, to any component which performs the specified function of the described component (e.g., that is functionally equivalent), even though not structurally equivalent to the disclosed structure which performs the function in the herein illustrated exemplary implementations of the disclosure. In addition, while a particular feature of the disclosure may have been disclosed with respect to only one of several implementations, such feature may be combined with one or more other features of the other implementations as may be desired and advantageous for any given or particular application. Furthermore, to the extent that the terms "includes," "having," "has," "with," or variants thereof are used in either the detailed description or the claims, such terms are intended to be inclusive in a manner similar to the term "comprising."

The implementations have been described, hereinabove. It will be apparent to those skilled in the art that the above methods and apparatuses may incorporate changes and modifications without departing from the general scope of this invention. It is intended to include all such modifications and alterations in so far as they come within the scope of the appended claims or the equivalents thereof.

## Claims

1. A device that applies torque to a target component and applies counter-torque to a base component while the target component is rotated with regard to the base component, the device comprising::
a power source portion at a proximal end of the device, the power source portion configured to generate torque;
an engagement portion at a distal end of the device, the engagement portion being selectably removable from the power source portion and including a counter-torque tube, a rotating shaft disposed within the counter-torque tube and configured to operably engage a target component and an engagement tip at the distal end of the counter-torque tube, the engagement tip configured to engage the base component; and
a torque limiter removably positioned between the power source portion and the engagement portion and configured to operably couple a powered distal tip of the power source portion and the rotating shaft,
wherein the torque limiter is configured to prevent a transfer of torque between the powered distal tip and the rotating shaft when the transferred torque is greater than a calibrated torque value.

2. The device of claim 1, wherein the torque limiter is configured to be a single-use component that is rendered useless by removal of the torque limiter from the device and disposed of after use in the device.

3. The device of claim 1 or claim 2, wherein the torque limiter comprises an input member and an output member, the input member axially received within a cavity of the output member, an outer wall of the input being configured to radially engage a cavity wall of the cavity.

4. The device of claim 3, the torque limiter further comprising a fastener configured to axially align and draw the input member and output member together, wherein fastening and loosening the fastener changes the calibrated torque value.

5. The device of claim 3 or claim 4, the input member comprising a plurality of input ridges equally distributed around the input member and extending outward and configured to engage a plurality of output ridges equally distributed around the cavity wall and extending inward from the cavity wall.

6. The device of claim 5, wherein each input ridge includes an increasing ramp, a plateau, and a decreasing ramp and each output ridge includes an increasing ramp, a plateau, and a decreasing ramp.

7. The device of any one of the preceding claims, the torque limiter including an output extension extending from a distal end of the torque limiter, the output extension including a groove defining a mechanical failure point if the applied torque exceeds the calibrated torque value by a predetermined threshold value.

8. The device of any one of the preceding claims, the engagement portion further comprising a display window that extends through a housing of the engagement portion and is configured to display an indicator on the torque limiter to provide visual confirmation of the calibrated torque value of the torque limiter.

9. The device of any one of the preceding claims, the engagement tip having a groove extending through a wall of the engagement tip, the groove having a load-bearing edge that is angled away from a centerline of the groove and is configured to engage a base component during fastening.

10. The device of any one of the preceding claims, further comprising a motor and a controller that is received within a handle of the power source portion, the motor configured to generate torque and the controller configured to:
calculate an applied torque based on a current draw of a battery that powers the device; and
reduce a speed of the motor when the applied torque approaches the calibrated torque value of the torque limiter.

11. The device of any one of claims 1 to 9, further comprising a motor and a controller that is received within a handle of the power source portion, the motor configured to generate torque and the controller configured to:
calculate an applied torque profile over time based on a current draw of a battery that powers the device; and
compare the calculated torque profile over time to an expected torque profile to determine if the target component is crossthreaded with the base component.

12. The device of any one of the preceding claims, wherein the torque limiter comprises, a cover plate, an input plate, and an output plate, the cover plate, input plate and output plate being axially aligned, wherein a resilient member is disposed between the cover plate and a top surface of the input plate to bias a bottom surface of the input plate into axially engagement with a top surface of the output plate.

13. The device of claim 12, the input plate comprising a plurality of input ridges equally distributed around the input plate and extending downward from the bottom surface of the input plate, the plurality of input ridges configured to engage a plurality of output ridges equally distributed around the output plate and extending upward from a top surface of the output plate.

14. The device of any one of the preceding claims, wherein the engagement tip is selectably removable from the counter-torque tube.

15. The device of any one of the preceding claims, wherein the torque limiter is retained on the distal end of the power source portion with a retaining device, and the device in configured such that a separate removal tool is used to remove the torque limiter, the removal tool configured to receive the torque limiter and engage the torque limiter such that a user can withdraw the removal tool to overcome the retaining device and remove torque limiter from the distal end of power source portion.
